# EUROPEAN PATENT APPLICATION

(11) **EP 4 194 490 A1**
(43) Date of publication of application: **14.06.2023**
(21) Application number: 21213470.4
(22) Date of filing: 09.12.2021
(51) Int. Cl.: C08J 11/16, C08J 11/24

(54) **A METHOD OF RECYCLING PLASTIC WASTE**

(71) Applicant: University College Cork-National University of Ireland Cork, Cork (IE); Loughborough University, Leicestershire LE11 3TU (GB)
(72) Inventor: Zangana, Karzan, Cork (IE); Fernandez-Mato, Antonio, Leicestershire, LE11 3TU (GB); Holmes, Justin, Cork (IE)
(74) Representative: Purdylucey Intellectual Property

(57) **Abstract**

A method of recycling a plastic product comprising PET is provided. The method comprises combining a plastic product comprising PET, a heterogeneous metal oxide catalyst and a solvent to provide a reaction mixture and heating the mixture by microwave irradiation to provide reaction product.

## Description

### Field of the invention

The current invention relates to a method for recycling plastic products, or materials, by depolymerisation. In particular, the current invention provides a method for recycling plastic products comprising or consisting of polyethylene terephthalate (PET).

### Background of the invention

The global demand for polymers (plastics) surpassed 275 million tonnes in 2019, with consumers using a mean of 50 products a day that depend on plastics for their functionality. Whilst the plastic industry is thriving, plastic pollution is causing global issues. Currently, 98 % of all plastic waste produced is non-biodegradable. A direct consequence of the disposal of this non-biodegradable plastic is the slow release of microplastics and synthetic fibres into the environment (Mar. Pollut. Bull., 2015, 93, 202).

Polyethylene terephthalate (PET) is a ubiquitous plastic commonly used in food packaging and 'fast fashion' clothing. Unfortunately, a significant amount of waste PET is disposed of by either burying in landfill sites or incineration. Many global brands are now working towards producing 100 % recyclable or compostable packaging by 2030. Recycled plastic materials are increasingly seen as a way to improve resource efficiency and the sustainable development of packaging. Europe is the second largest regional market for plastic recycling, accounting for almost a quarter of the global recyclate production and consumption in 2019. Mechanical recycling is the most widely used approach for treating plastic waste, such as polyethylene terephthalate (PET) but is considered only a temporary solution, as after each cycle the economic value of the plastic is reduced (downcycling) due to the loss of the properties of the polymer relative to the virgin material.

Chemical recycling, although not currently widely used, has the potential to be able to recycle "mixed" plastic waste and more difficult to recycle packaging (Nature Rev Chem., 2017, 1, 0046). Chemical recycling could solve the problem of downcycling by retaining the value of the monomers in plastics such as PET. However, current methods of chemical recycling, such as the widely studies glycolysis of PET, are often energy intensive and employ homogeneous catalysts that are difficult to recover and recycle, reducing chemical recycling as an economic and environmental viability process. Consequently, current chemically technologies for recycling PET cannot compete in price with virgin PET.

EP3377569 discloses a method of chemical recycling of PET using microwave as an energy source for the depolymerisation of PET. This process uses a homogeneous catalyst and diethylene glycol as a solvent. The homogeneous catalyst is difficult to recover and reuse.

EP2736968 discloses a method and apparatus for the recycling of polymeric materials via a microwave depolymerisation process. The process described is a continuous process for the depolymerisation of ester polymers, including PET, using microwave and alkaline salts such as NaOH and KOH as homogeneous catalysts. Terephthalic acid or the salts of terephthalic acid are obtained. However, the subsequent neutralisation of the basic reaction conditions is not economical from the industrial perspective.

Song et al. (Journal of Applied Polymer Science, Vol. 117,3155-3159 (2010)) discloses a method of chemical recycling using metal oxide catalysts (MgO, Fe₂O₃, Gr₂O₃, ZnO, SnO, Sb₂O₃, Cu₂O, TiO₂, and MnO₂), microwaves and water as solvent for PET hydrolytic depolymerisation. Therefore, this process does not use glycolysis and the product obtained is terephtalic acid (TPA). This process also requires waste solvent to be neutralised.

Siraphat Putisompon, et al (Key Engineering Material, Vol. 824, pp 225-230, 2019) discloses a method of chemical recycling using CaO as a catalyst with ethylene glycol as a solvent for PET depolymerisation using thermal heating with a depolymerisation time of two hours. CaO catalysts derived from shells were used in this reaction. After the reaction, the product is precipitated by adding water to the mixture with ethylene glycol. Therefore, this process does not allow the reuse of ethylene glycol, unless purified by distillation, and due to the presence of water, the catalyst cannot be recovered.

The current invention solves to alleviate the above problems.

### Summary of the invention

The current invention provides a method for recycling plastic products, such as low-value polyester and PET sources, e.g., mixed plastics, textiles, carpet fibres or similar, and the optional subsequent conversion of the chemical species formed during the recycling process into plastics of higher value (upcycling). The method involves depolymerising PET into its basic components (monomers) and optionally re-polymerising the components back into PET or into plastics of higher value. These higher value plastics may be polybutylene adipate terephthalate (PBAT) and polybutylene terephthalate (PBT).

In a first aspect, the current invention provides a method for recycling (depolymerisation) a plastic product, the method comprising:
combining a plastic product, a heterogeneous metal oxide catalyst and a solvent to provide a reaction mixture,
heating the reaction mixture by microwave irradiation to provide a reaction product comprising a depolymerisation product.

Preferably, the plastic product comprises polyethylene terephthalate (PET). The plastic product may consist of PET.

The method is one of depolymerisation and the reaction product comprises, or consists of, the depolymerisation product, i.e., monomers.

Preferably, the catalyst is a heterogenous metal oxide, preferably, calcium oxide (CaO) or zinc oxide (ZnO).

Typically, the heterogenous metal oxide is CaO.

Preferably, the catalyst is nanostructured.

Preferably, the solvent is selected from ethylene glycol and butylene diol (1, 4-butanediol).

Typically, the method further comprises a step of recovering or purifying the depolymerisation product. This may be a step of crystallising the depolymerisation product Preferably, the method further comprises a step of solvent recovery and/or a step of catalyst recovery. This may be by filtration.

In an embodiment, the catalyst is dispersed in the solvent prior to addition of the plastic product. Preferably, the catalyst and solvent are stirred and/or agitated prior to adding the plastic product.

Typically, the plastic product is PET or a PET containing product.

In an embodiment, the solvent is ethylene glycol, and the reaction product comprises BHET.

In an embodiment, the solvent is 1, 4-butanediol, and the reaction product comprises BHBT.

In an embodiment, the reaction mixture may optionally comprise a microwave absorber. The microwave absorber may be selected form the group comprising sodium chloride, sodium bromide, sodium iodide, sodium fluoride, lithium chloride, potassium chloride, magnesium chloride, calcium chloride and activated charcoal.

An aspect of the current invention provides a method of converting PET into bis-hydroxy ethylene terephthalate (BHET), the method comprising
combining a PET or a PET containing plastic product, a heterogeneous metal oxide catalyst and ethylene glycol to provide a reaction mixture,
heating the reaction mixture by microwave irradiation to produce BHET.

An aspect of the current invention provides a method of converting PET into bis-hydroxy butylene terephthalate (BHBT), the method comprising
combining a PET or a PET containing plastic product, a heterogeneous metal oxide catalyst and butylene diol,
heating the reaction mixture by microwave irradiation to provide BHBT.

### Definitions and General Preferences

Where used herein and unless specifically indicated otherwise, the following terms are intended to have the following meanings in addition to any broader (or narrower) meanings the terms might enjoy in the art:

Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or open-ended and does not exclude additional, unrecited integers or method/process steps.

"Recycling" is the process of converting materials, such as plastic waste materials, into new materials and objects. Ideally the new material is a reusable material.

As used herein the term "depolymerisation" refers to a process of converting a polymer into a monomer or a mixture of monomers. The monomer(s) is the "depolymerisation product"

As used herein the term "heterologous catalyst" refers to a catalyst in a phase different form that of the reactants in a reaction.

As used herein the term "nanostructured" refers to a material that has a size of 1 to 100 nm.

As used herein the term "polyethylene terephthalate (PET), is a thermoplastic resin. It is commonly used in fibres for clothing and containers such as plastic bottles. It consists of polymerised units of the monomer ethylene terephthalate with repeating C₁₀H₈O₄ units. In this context, PET may be provided as PET or as a plastic product comprising PET. It has the following chemical structure:

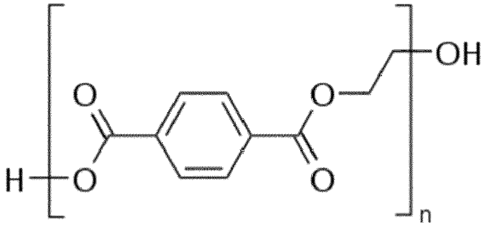

As used herein the term "crystallisation" refers to the process of producing solid crystals from a solution. In general, a solvent evaporates leaving a concentrated solution.

As used herein the term "plastic product" refers to a synthetic, semi-synthetic or naturally occurring material comprising a polymer as the main component. Preferably, the polymer is PET. The plastic product may be any known plastic product, such as a waste product, or container. Examples include but are not limited to textiles, bottles, cosmetic containers and food containers. The plastic product may be at least one plastic product, i.e., a combination of plastic products.

As used herein the term "microwave irradiation" is a form of electromagnetic radiation with wavelengths ranging from about 1 metre to one millimetre corresponding to frequencies between 300 MHz and 300 GHz respectively.

"Ethylene glycol" is an organic compound with the formula (CH₂OH)₂.

"1,4-Butanediol" is an organic compound with the formula C₄H₁₀O₂ or HO(CH₂)₄OH

As used herein the term "catalyst support" is a material, usually with a high surface area, to which a catalyst is affixed. A "supported catalyst" is a catalyst with a support.

### Brief Description of the Figures

The current invention will be more clearly understood from the following description of an embodiment thereof, given by way of example only, with reference to the accompanying Figures in which;
**Figure 1** is a simplified representation of the chemical process described for the depolymerisation process of the current invention
**Figure 2** shows chemical pathways for the depolymerisation of polyester fabrics using ethylene glycol to form BHET crystals and butylene diol to form BHBT.

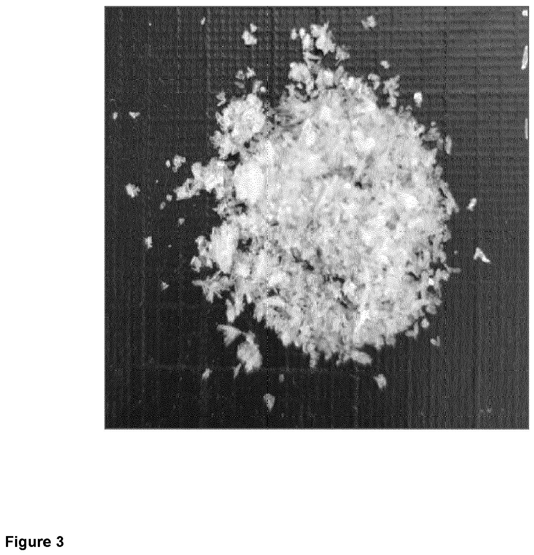

**Figure 3** illustrates BHET crystals formed from the glycolysis of polyester fabrics.

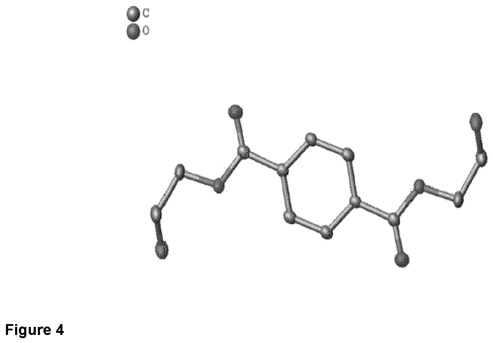

**Figure 4** illustrates the crystal structure of BHET obtained from X-ray diffraction data.
**Figure 5** illustrates the schemes for the glycolysis reaction using 1,4-butanediol solvent and using ethylene glycol as a solvent.

### Detailed description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

The method of the invention uses the combination of a heterologous metal oxide catalyst and a microwave energy source to depolymerise PET into its basic components (monomers) and optionally re-polymerise the components back into PET or into plastics of higher value.

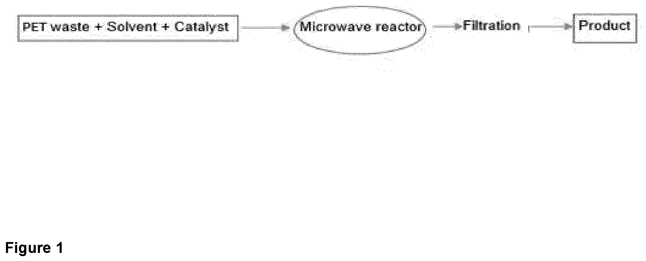

Figure 1 is a simplified representation of the chemical process described for the depolymerisation process of the current invention. As illustrated, the method involves combining the plastic product, a heterologous nanostructured metal oxide catalyst and a solvent to provide a reaction mixture. The plastic product and substrate are heated by microwave radiation in the presence of the heterologous catalyst.

In an embodiment, the plastic product is processed prior to addition to the reaction mixture. For example, it may be washed and/or cleaned. It may be broken, or cut, crushed and/or shredded. These types of processing steps prior to recycling are known in the art.

The solvent and catalyst maybe combined and optionally stirred and/or agitated, prior to the addition of the plastic product to the reaction mixture.

Typically, the reaction mixture is agitated or stirred prior to exposing to the energy source.

Of note, the current invention employs a heterogeneous metal oxide catalyst, such as CaO or ZnO, MgO or Iron oxides. Iron oxide may be Iron (III) oxide, namely Fe₂O₃. It may be FeO or Fe₃O₄. It may be a mixture of one or more of the catalysts. In an embodiment, the catalyst may be nanostructured. Typically, the catalyst is dispersed in the solvent. Advantageously, the catalyst used in the current invention can be easily separated and recovered, thus reducing the amount of purification steps required for the isolation of the product after PET depolymerisation. The catalyst is also easily regenerable or reusable. No aqueous treatment step is necessary when using a heterogenous catalyst.

The catalyst may be a supported catalyst, for example supported CaO catalyst. Supports are known in the art. Examples of supported include but are not limited to activated carbon and activated charcoal. This applies to any one of the catalysts disclosed herein. Examples of supported catalysts include but are not limited to supported CaO/MgO, activated carbon supported CaO, MgO and iron oxides, nano CaO, CaO on AC (activated carbon and activated charcoal) supports, Fe₃O₄/AC, ZnO/CaO mixture, MgO/AC, ZnO/AC and Fe₂O₃/AC.

In a typical reaction, the catalyst is included in the reaction mixture in a concentration of 0.1 % to 2 %, typically 1 % by weight of PET, or 1.5% by weight of PET.

The plastic product is included in the reaction mixture in any suitable amount, e.g., from 0.5g to 10g PET, from 1 to 5g PET, typically 1 to 2g PET.

Plastic product, e.g., PET, and solvent are used in a 10:1 ratio (w/w%). The ratio may be 20:1, 15:1, 10: 1, or 5:1.

In a preferred embodiment, the reaction mixture comprises 1g of PET, 0.05g of catalyst and 10ml of solvent.

Notably, the current invention uses microwave as an energy source. This substantially reduces the amount of time and energy required compared with prior art methods which use thermal heating, with the use of a cheap and non-toxic catalyst that can be easily separated and recovered. For example, using a CaO catalyst together with microwave energy, the depolymerisation of a plastic comprising PET can take less than 3 minutes. Using the same catalyst with thermal heating can take two hours for depolymerisation.

The temperature of the microwave irradiation is one sufficient to produce a reaction product comprising a depolymerisation product. It is preferably at least, or at, the boiling point of the solvent. For example, when ethylene glycol is used as a solvent, the heating step by microwave energy is from 190 °C to 200 °C, preferably about 197 °C. When 1, 4-butanediol is the solvent the temperature is from 225 °C to 235 °C, preferably about 230 °C.

The time of this step is one suitable for the depolymerisation reaction to complete. For example, 30 minutes or less, 25 minutes or less, 20 minutes or less, 15 minutes or less, 12 minutes or less, 10 minutes or less, 10 minutes or less, 9 minutes or less, 8 minutes or less, 7 minutes or less, 6 minutes or less, 5 minutes or less, 4 minutes or less, 3 minutes or less, or 2 minutes or less. Typically, it is from 1 to 30 minutes, from 5 to 25 minutes, from 10 to 15 minutes. It will be appreciated that the time depends on the plastic product used.

### Following irradiation or exposure to the heating source,

The produced product, or depolymerisation product, obtained depends on the solvent used and this can be manipulated or tuned by the user by changing the type of solvent used in the method of the invention. For example, when ethylene glycol is used as a solvent bis-hydroxy ethylene terephthalate (BHET) is the predominant depolymerisation product produced. This method uses glycolysis for depolymerisation. When butylene diol is used as a solvent, the predominant product produced is bis-hydroxy butylene terephthalate (BHBT). This method also uses glycolysis for depolymerisation.

The depolymerisation product can subsequently be polymerised, or re-polymerised, into a higher value polymer, e.g., PET. The type of product produced depends on the starting depolymerisation product. BHET can subsequently be used to regenerate PET and BHBT can be used to produce biodegradable polymers such as PBT or PBAT. PBAT is a promising candidate as a biodegradable packing material and could be used in products such as food grade plastic bottles. Thus, the method of the invention may further comprise a step of polymerisation of the depolymerised product to generate polymer. Methods of polymerisation are known in the art. For example, Denial Mahata, et al., (Poly(butylene adipate-co-terephythalate) polyester synthesis process and product development. Polym. Sci. Ser. C 63, 102-111 (2021)) and H Shah, et al., (Aspects of the chemistry of poly(ethylene terephthalate): 5. Polymerization of bis(hydroxyethyl)terephthalate by various metallic catalysts, Polymer, volume 25, issue 9,1984).

When the depolymerisation product is produced, such as BHET, advantageously no extra separation steps are required for isolating the product, such as distillation used in conventional chemical recycling processes. Instead, high purity BHET is provided. This may be by any known means. The monomer product, e.g., BHET monomer product, is recovered or purified. This may be a means for recovery known in the art. It includes a step of crystallisation or extraction. Extraction may be by using solvents such as chloroform or dichloromethane DCM. Preferably, the step of recovery is by crystallisation.

After depolymerisation the reaction and prior to purification/recovery, the reaction is normally cooled to stop the reaction. This may be to approximately 100°C. This can be by any means known in the art, e.g., by leaving it at room temperature. Preferably, it is cooled using a water bath.

In the current method, preferably purification/recovery is by crystallisation in solution. Generally, it is crystallised from the mother liquor with a with a purity of > 95 % as determined by nuclear magnetic resonance (NMR) analysis. Methods of NMR are known in the art. For example, Ritter Lima et al., (Titanate nanotubes as new nanostructured catalyst for depolymerisation of PET by glycolysis reaction., Articles Mat. Res. 20 (suppl 2) 2017).

This crystallisation step reduces both solvent use and energy used in the overall process, compared with conventional chemical recycling which extract the product using solvents.

In an embodiment, the purity of the depolymerisation product is equal to or greater than 80 %, 85 %, 90 %, 95 %, or 98 %, or 100 %. Typically, it is between 90 % and 100 %, or from 95 % to 98 %. Preferably, the purity is greater than 95%.

The recovered product can then be separated by any known means form the mother liquor. Typically, separation is by filtration.

The method comprises a step of solvent recovery and/or a step of catalyst recovery. This may be by means for recovery known in the art. It includes filtration. Typically, this step is after purification/recovery of the depolymerisation product, e.g., after crystallisation.

Alternatively, the catalyst recovery system could be an aqueous/organic mixture. In such a means, the catalyst is dissolved in the aqueous phase and the reagents are in the organic phase. Distillation and condensation can be used to separate and recover solvent from other liquids. Removal efficiency can be very high using this type of process and can be used for solvent mixtures as well as single solvents.

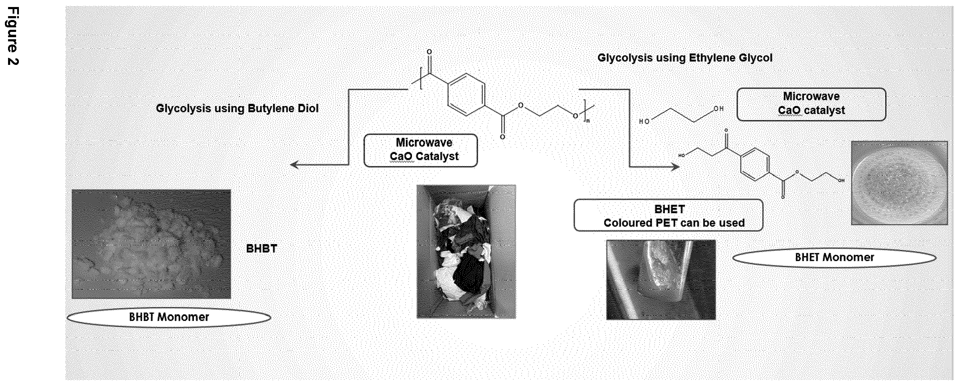

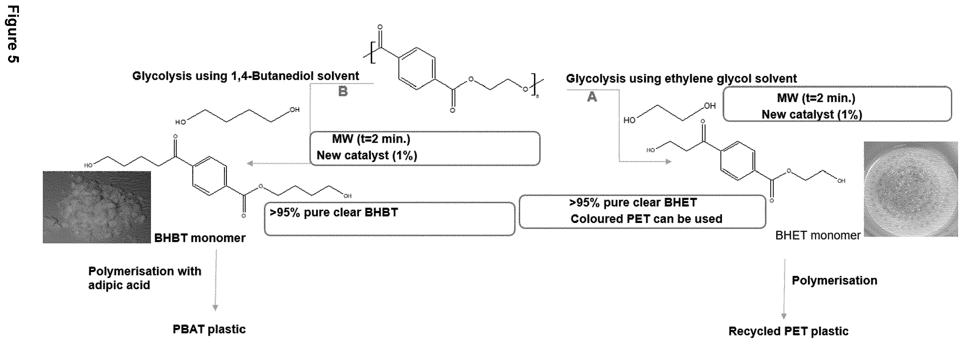

Figure 2 shows chemical pathways for the depolymerisation of polyester fabrics using ethylene glycol to form BHET crystals and butylene diol to form BHBT. Both are undertaken by glycolysis to produce the monomers. Figure 5 illustrates the reaction scheme for glycolysis using 1,4-butanediol solvent and using ethylene glycol as a solvent.

In the method of the current invention, the PET may be coloured PET. Coloured PET can be depolymerised without interfering with the crystallisation process. Using coloured PET for recycling can cause several issues, such as the purity of the final product. Usually, further steps of purification are needed when a coloured plastic is used as the starting product. However, the current method can provide pure (colourless) crystals from coloured mother liquor. This is important to reduce the cost of recycling and prevent the use of hazardous solvents.

In an embodiment, the reaction mixture may optionally comprise a microwave absorber or microwave absorbing material. The microwave absorber may be selected form the group comprising sodium chloride, charcoal, activated charcoal, sodium bromide, sodium iodide, sodium fluoride, lithium chloride, potassium chloride, magnesium chloride and calcium chloride. This functions to dissipate electromagnetic waves by converting it into thermal energy.

The method of the current invention provides a conversion of equal or greater than 75 %, equal or greater than 80 %, equal or greater than 85 %, equal or greater than 90 %, equal or greater than 95 %, equal or greater than 98 %, or 100 %. Typically, the conversion is equal or greater than 80 %, or from 80 % to 90 %, or 80 % to 95 %. The method of the current invention provides a yield of greater than or equal to 80%.

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### EXAMPLE 1

### Depolymerisation Procedure

### Methodology

One gram (1g) of PET was added into 100 ml round bottom flask and followed by the 0.1 g of CaO catalyst to PET. Ethylene glycol was added into a round bottom flask and stirred for mixing all starting materials. This mixture was heated to the boiling point of ethylene glycol (-200 °C) in a microwave, the glycolysis reaction was allowed to proceed for 2 to 4 min, after which time the temperature of the solution lowered to about 100 °C in a water bath to stop the reaction. The hot solution of BHET was filtered by filtration. The filtrate cooled down to room temperature to obtain a precipitate of BHET. The products formed from the depolymerisation of PET were characterised by NMR and X-ray diffraction analysis.

### Results

¹H NMR data of the products obtained can be summarised as follows: protons of an aromatic ring (δ_{H} = 8.1 ppm, s, 4H), hydroxyl groups (δ_{H} = 4.95 ppm, t, 2H), methylenes (-CH₂-) adjacent to the -OH groups (δ_{H} = 3.73 ppm, m, 4H), methylenes (-CH₂-) adjacent to the -COO groups (δ_{H} = 4.33 ppm, t, 4H), indicative of the formation of BHET. A signal observed at approximately 2.5 ppm can be assigned to the solvent DMSO and peaks at 3.3 and 2.0 ppm were attributed to residual H₂O and contamination. ¹³C NMR data of the products showed the following peaks: (δ_{c} = 165.65 ppm), (δ_{c} = 134.24 ppm), (δ_{c} = 129.96 ppm), (δ_{c} = 67.48 ppm) and (δ_{c} = 59.48 ppm), which can be assigned to the carbons of the chemical structure of BHET. The signal from DMSO appeared at 40 ppm.

X-ray diffraction data of the crystals obtained from the glycolysis of polyester fibres in ethylene glycol also confirmed the presence of BHET as the major product formed (see Table 1).

**Table 1: X-ray crystallography data obtained from a BHET crystal formed from the glycolysis of polyester fibres in ethylene glycol.**

| Crystallographic information for BHET | |
|---|---|
| Empirical formula | C₄₈H₅₆O₂₄ |
| Formula weight | 1016.97 |
| Temperature/K | 296.15 |
| Crystal system | monoclinic |
| Space group | P2₁/c |
| a/Å | 8.4390(15) |
| b/Å | 5.4417(10) |
| c/Å | 25.567(5) |
| α/° | 90 |
| β/° | 98.046(3) |
| γ/° | 90 |
| Volume/Å³ | 1162.5(4) |
| Z | 1 |
| ρ_{calc}g/cm³ | 1.4525 |
| µ/mm⁻¹ | 0.118 |
| F(000) | 536.4 |
| Crystal size/mm³ | 0.65 x 0.25 x 0.14 |
| Radiation | Mo Kα (λ = 0.71073) |

### EXAMPLE 2

### Depolymerisation Procedure

### Methodology and Results

To a 50 ml flask containing CaO (0.1 g) was charged 1,4- butanediol (10 ml) and PET (1 g). The flask was heated using microwave energy with stirring C for 3 minutes. The slurry turned into a homogeneous liquid in 3 minutes. The reaction mixture was dissolved in chloroform (50 mL) and washed with water (100 mL). The organic layer was then stirred, evaporated, and dried in vacuum to give the product bis(4-hydroxybutyl) terephthalate (BHBT) (0.9 g, 90%).

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method for recycling a plastic product comprising PET, the method comprising
combining a plastic product comprising PET, a heterogeneous metal oxide catalyst and a solvent to provide a reaction mixture,
heating the reaction mixture by microwave heating to provide a reaction product comprising a depolymerisation product.

2. The method of Claim 1, wherein the catalyst is a heterogenous nanostructured metal oxide.

3. The method of Claim 1 or 2, wherein the catalyst is calcium oxide (CaO), zinc oxide (ZnO), Magnesium oxide (MgO) or iron oxide (Fe₂O₃)

4. The method of Claim 3, wherein the catalyst is CaO.

5. The method of any one of the preceding claims, wherein the catalyst is a supported catalyst.

6. The method of any one of the preceding claims wherein the solvent is selected from ethylene glycol and 1, 4-butanediol.

7. The method of any one of the preceding claims, further comprising a step of recovering the depolymerisation product from the reaction product.

8. The method of Claim 7, wherein the step of recovering the depolymerisation product is by crystallisation.

9. The method of any one of the preceding claims, further comprising a step of solvent recovery and/or a step of catalyst recovery.

10. The method of any one of the preceding claims, wherein the reaction mixture comprises a microwave absorber.

11. The method of Claim 10, wherein the microwave absorber may be selected form the group comprising sodium chloride, sodium bromide, sodium iodide, sodium fluoride, lithium chloride, potassium chloride, magnesium chloride, calcium chloride and activated charcoal.

12. The method of any one of the preceding claims, further comprising a step of polymerisation of the depolymerisation product.

13. A method of converting PET into bis-hydroxy ethylene terephthalate (BHET), the method comprising
combining PET or a PET containing product, a heterogeneous metal oxide catalyst and ethylene glycol to provide a reaction mixture,
heating the reaction mixture by microwave heating to produce BHET.

14. A method of converting PET into bis-hydroxy butylene terephthalate (BHBT), the method comprising
combining a PET or a PET containing product, a heterogeneous metal oxide catalyst and butylene diol,
heating the reaction mixture by microwave heating to provide BHBT.
